(19) Europäisches Patentamt
European Patent Office
Office européen des brevets

(11) **EP 4 748 393 A1**

(12) **EUROPEAN PATENT APPLICATION**
published in accordance with Art. 153(4) EPC

(43) Date of publication:
**27.05.2026 Bulletin 2026/22**

(21) Application number: **24835421.9**

(22) Date of filing: **05.07.2024**

(51) International Patent Classification (IPC):
**A61K 39/395** (2006.01)    **A61P 9/00** (2006.01)

(52) Cooperative Patent Classification (CPC):
**A61K 39/395; A61P 9/00**

(86) International application number:
**PCT/CN2024/103739**

(87) International publication number:
**WO 2025/007943 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.07.2023 CN 202310821860**

(71) Applicant: **RemeGen Co., Ltd.**
**Yantai, Shandong 264006 (CN)**

(72) Inventors:
• **FANG, Jianmin**
**Shandong 264006 (CN)**
• **WANG, Wenxiang**
**Shandong 264006 (CN)**
• **WU, Jingping**
**Shandong 264006 (CN)**

(74) Representative: **Cooley (UK) LLP**
**22 Bishopsgate**
**London EC2N 4BQ (GB)**

Remarks:
The complete document including Reference Table(s) and the Sequence Listing(s) can be downloaded from the EPO website

(54) **METHOD FOR TREATING ANCA-ASSOCIATED VASCULITIS USING TACI-FC FUSION PROTEIN**

(57)    A drug for treating ANCA-associated vasculitis (AAV) using an effective amount of a drug targeting BLyS and/or APRIL, a dosage regimen, a dosing interval, and an administration mode. The results show that the provided effective amount of a drug targeting BLyS and/or APRIL demonstrates good safety and therapeutic effects in the treatment or alleviation of patients with ANCA-associated vasculitis.

FIG. 1

EP 4 748 393 A1

## Description

## Technical Field

The present invention relates to a TACI-Fc fusion protein drug for treating ANCA-associated vasculitis, a dosage regimen, a dosing interval, and an administration mode.

## Related Art

Anti-neutrophil cytoplasmic antibody (ANCA)-associated vasculitis (AAV) is an autoimmune disease characterized by necrotizing vasculitis of small and medium-sized blood vessels. It includes three disease types: granulomatosis with polyangiitis (GPA), eosinophilic granulomatosis with polyangiitis (EGPA, formerly known as Churg-Strauss syndrome), and microscopic polyangiitis (MPA) (Reference 1: Gapud E J, Seo P, Antiochos B. ANCA-associated vasculitis pathogenesis: a commentary[J]. Current rheumatology reports, 2017, 19: 1-7).

ANCA-associated vasculitis can occur in all age groups, with a prevalence of approximately 46-184 cases per million people. The incidence and types of AAV vary across different regions. In recent years, the global incidence of ANCA-associated vasculitis has been gradually increasing. Specifically, the prevalence of granulomatosis with polyangiitis (GPA) is about 1 in 25,000, the prevalence of microscopic polyangiitis (MPA) is approximately 1-3 per 100,000 people, and the prevalence of eosinophilic granulomatosis with polyangiitis (EGPA) is about 10.7-13 per 1,000,000 people. Overall, the increase in the prevalence of ANCA-associated vasculitis can be attributed to factors such as rising incidence, improved disease criteria, increased/expanded databases, and increased survival rates.

ANCA-associated vasculitis is typically fatal if left untreated, with a 6-month mortality rate of about 60% and a 1-year mortality rate as high as 80%. The disease mainly involves small blood vessels and is pathologically characterized by full-thickness inflammation and necrosis of small vessels, with or without granuloma formation. Common symptoms may include fever, arthralgia, myalgia, fatigue, and weight loss. When the lungs, kidneys, gastrointestinal tract, nervous system, or eyes, ears, nose, and throat are involved, corresponding clinical manifestations appear. Systemic musculoskeletal symptoms, although non-specific, are common. The following clinical manifestations may also occur alone or in combination: chronic recurrent sinusitis accompanied by nasal crusting, cavitary lung nodules, rapidly progressive necrotizing pauci-immune complex glomerulonephritis, mononeuritis multiplex, pericarditis or myocarditis, and obvious purpura. Acute severe ANCA-associated vasculitis can present with simultaneous respiratory and renal failure (i.e., "pulmonary-renal syndrome"). Other studies have shown that compared with the general population, the risk of malignant tumors in patients with ANCA-associated vasculitis is significantly increased, with a standardized incidence rate of 1.74.

The treatment of ANCA-associated vasculitis is mainly divided into two phases: an induction of remission treatment phase and a maintenance of remission treatment phase. The treatment cycle of the induction of remission treatment phase is generally 3-6 months. In this phase, patients require immediate treatment to rapidly alleviate inflammation to prevent death and limit permanent organ damage. The treatment cycle of the maintenance of remission treatment phase is generally 24-48 months. In this phase, patients generally show no signs of disease but require treatment to prevent recurrence. Although treatment during the induction of remission treatment phase has transformed ANCA-associated vasculitis from a life-threatening disease into a chronic disease, recurrence is very common, and 30%-50% of patients usually experience recurrence within 12-18 months after stopping immunosuppressive therapy. Overall, therapeutic drugs for ANCA-associated vasculitis are currently mainly divided into the following categories: glucocorticoids, immunosuppressants (such as azathioprine, cyclophosphamide, mycophenolate mofetil, etc.), and biological agents (such as rituximab, mepolizumab), etc.

① Glucocorticoids (GC): Glucocorticoids play a central role in the treatment of ANCA-associated vasculitis and are a necessary component of initial treatment, especially when there is renal involvement, but glucocorticoids alone cannot sustainably induce remission. In addition, glucocorticoids have widespread side effects, including infection, bone disease, blood glucose abnormalities, obesity, hypertension, psychiatric disorders, gastrointestinal bleeding, cataracts, adrenal suppression, and long-term risks of cardiovascular diseases. In early studies using glucocorticoids to treat patients with ANCA-associated vasculitis, reported common adverse events included: weight gain >10 kg (29%), newly diagnosed diabetes (8.2%), peptic ulcer disease (2.6%), fractures (2.5%), and avascular necrosis (0.4%). During long-term follow-up, cataracts (25%), diabetes (38%), osteoporosis (38%), and hypertension (41%) occurred. The severity and frequency of these adverse reactions have also limited the use of glucocorticoids.

② Immunosuppressants: ANCA-associated vasculitis with organ involvement and life-threatening manifestations can be treated with a combination of glucocorticoids and cyclophosphamide (CTX). Although the effectiveness of this regimen is >90%, it is limited by its potential toxicity. Studies have shown that CTX is associated with a variety of severe adverse reactions; many occur in the early stage (such as myelosuppression, infection, hemorrhagic cystitis, and infertility), while others may occur 10 years or even longer after the end of immunosuppressive therapy (such as malignant tumors).

③ Biological agents: Currently, 3 biological agents have been approved for the treatment of ANCA-associated vasculitis, namely rituximab, mepolizumab, and avacopan. Rituximab (RTX) is a chimeric anti-CD20 monoclonal antibody. In April 2011, the U.S. FDA approved RTX as an alternative to cyclophosphamide (CTX) combined with glucocorticoids for the treatment of severe GPA/MPA. Although the mechanism of RTX in patients with ANCA-associated vasculitis is not fully understood, it can reduce ANCA-producing B cells and plasmablasts, thereby treating the disease. For Asian or Chinese patients, the use of recommended doses also carries a high possibility of infection.
In a study involving AAV patients, patients received RTX treatment at a dose of 375 mg/m$^2$ RTX injected weekly for 4 weeks; or 1 g of RTX injected twice a month. During follow-up, 37% of patients developed severe infections. Evidence suggests that the administration of corresponding doses of RTX carries a risk of similar adverse reactions; 50% of patients developed infections after treatment, which may be attributed to the use of excessively high doses (Reference 3: Liu L, Lu H, Zou G, et al. Efficacy and safety of low-dose rituximab as induction therapy for antineutrophil cytoplasmic antibody-associated vasculitis with renal involvement: a Chinese case series. BMC Nephrol. 2023 Feb 8;24(1):28).

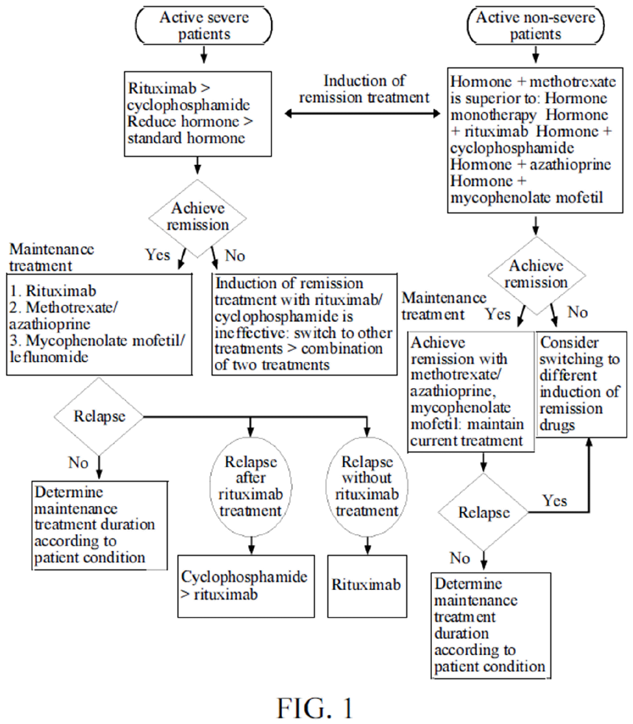

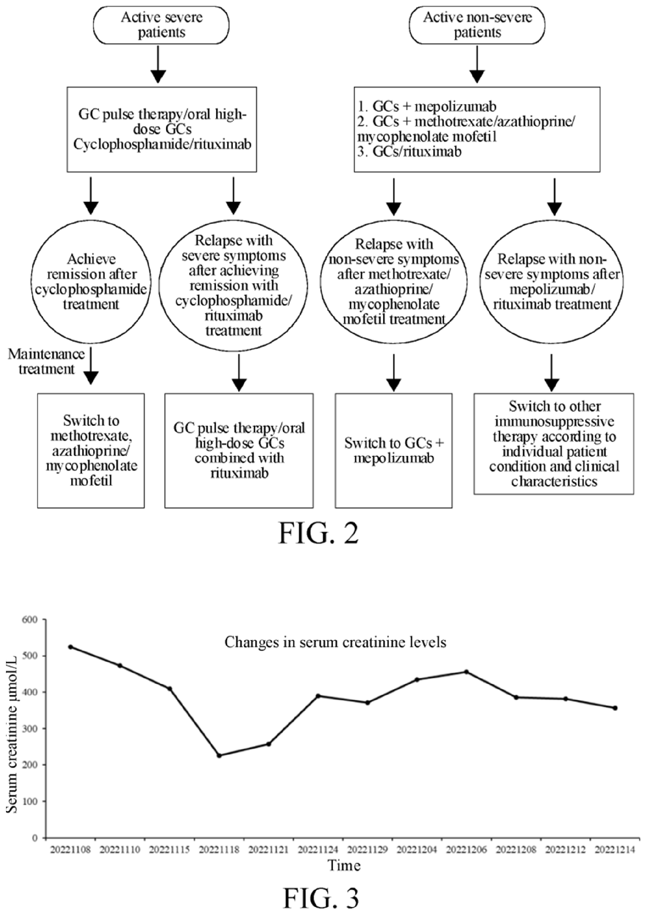

The American College of Rheumatology (ACR) first developed management guidelines for ANCA-associated vasculitis in 2021, providing guidance recommendations for the treatment of granulomatosis with polyangiitis (GPA) and microscopic polyangiitis (MPA) (see FIG. 1) and eosinophilic granulomatosis with polyangiitis (EGPA) (see FIG. 2), respectively (Reference 2: Chung, S. A., Langford, C. A., Maz, M., Abril, A., Gorelik, M., Guyatt, G., ...Mustafa, R. A. (2021). 2021 American College of Rheumatology/Vasculitis Foundation Guideline for the Management of Antineutrophil Cytoplasmic Antibody-Associated Vasculitis. Arthritis Care & Research, 73(8), 1088-1105.).
However, the level of evidence for the above guidance recommendations is generally low. It remains imperative to conduct more studies on ANCA-associated vasculitis to provide more targeted drugs and guidance for disease treatment, minimize treatment side effects, and prevent organ damage in patients. Therefore, both in China and globally, there remains a realistic unmet clinical need in the treatment of ANCA-associated vasculitis. Particularly for patients who are unable or unwilling to use long-term hormone therapy due to various diseases, there is a lack of safe and effective clinical therapeutic drugs.
Belimumab is a BLyS-specific inhibitor that can bind to BLyS and block its binding to receptors on B cells, thereby inhibiting the survival of autoreactive B cells and allowing more autoreactive B cells to undergo apoptosis. However, according to the introduction in the 2020 French Vasculitis Study Group Treatment Guidelines, the expert group does not recommend the use of belimumab (i.e., belimumab) for the induction therapy of GPA or MPA. A study showed that compared with placebo, belimumab did not reduce the risk of PSE (protocol-specified events) or vasculitis recurrence. The rate of adverse events in the belimumab group was significantly higher than that in the placebo group (92.5% vs 82.7%). The use of belimumab plus azathioprine and glucocorticoids to maintain AAV remission also did not reduce the risk of recurrence (Reference 4: David, Jayne, Daniel, et al. Efficacy and Safety of Belimumab and Azathioprine for Maintenance of Remission in Antineutrophil Cytoplasmic Antibody-Associated Vasculitis: A Randomized Controlled Study. Arthritis & rheumatology (Hoboken, N.J.), 2019.). This may be related to the expert group's recommendation against the use of belimumab for the induction therapy of GPA or MPA.
Telitacicept is a first-in-class recombinant TACI-Fc fusion protein targeting B cell-related autoimmune diseases. It can target and neutralize two key cellular signaling molecules of the B cell pathway, BLyS and APRIL. It is an antibody-like structural fusion protein for the treatment of human autoimmune system diseases, consisting of a truncated TACI and an immunoglobulin Fc with ADCC and CDC effects reduced after sequence optimization. It possesses excellent biological activity and safety and has currently been approved for marketing in China for the treatment of systemic lupus erythematosus.

## SUMMARY

However, the present invention surprisingly found that the TACI-Fc fusion protein exhibits excellent biological activity and safety when treating patients with ANCA-associated vasculitis, resulting in a significant therapeutic effect.
To this end, the present invention provides a method for treating or alleviating ANCA-associated vasculitis, including administering a therapeutically effective amount of a drug targeting BLyS and/or APRIL to a patient having the ANCA-associated vasculitis.
The present invention also provides an application of a drug targeting BLyS and/or APRIL in preparation of a medicament for treating or alleviating ANCA-associated vasculitis in a patient.
The present invention also provides an application of a TACI-Fc fusion protein in preparation of a medicament for treating or alleviating ANCA-associated vasculitis in a patient.

The present invention also provides an application of telitacicept in preparation of a medicament for treating or alleviating ANCA-associated vasculitis in a patient.

Further, the drug targeting BLyS and/or APRIL described above is a TACI-Fc fusion protein.

Further, the TACI-Fc fusion protein according to any one of the above includes:

(i) an extracellular domain of TACI or a fragment thereof binding to BLyS and/or APRIL; and

(ii) a human immunoglobulin constant region fragment.

Further, the extracellular domain of TACI or the fragment thereof binding to BLyS and/or APRIL includes an amino acid sequence shown in SEQ ID NO: 1, SEQ ID NO: 2, or SEQ ID NO: 3.

SEQ ID NO:1

```
SRVDQEERFP QGLWTGVAMR SCPEEQYWDP LLGTCMSCKT ICNHQSQRTC AAFCRSLSCR      60
KEQGKFYDHL LRDCISCASI CGQHPKQCAY FCENKLRSPV NLPPEL                    106
```

SEQ ID NO:2

```
AMRSCPEEQY WDPLLGTCMS CKTICNHQSQ RTCAAFCRSL SCRKEQGKFY DHLLRDCISC      60
ASICGQHPKQ CAYFCENKLR S                                                81
```

SEQ ID NO:3
SLSCRKEQGE YYDHLLRDCI SCASICGQHP KQCADFCENK LRS 43

Further, the amino acid sequence of the extracellular domain of TACI or the fragment thereof binding BLyS and/or APRIL is as shown in SEQ ID NO: 1.

Further, a human immunoglobulin is IgG1.

Further, the human immunoglobulin constant region fragment includes an amino acid sequence of SEQ ID NO: 4.

Further, the human immunoglobulin constant region fragment includes an amino acid sequence having at least 90%, at least 91%, at least 92%, at least 93%, at least 94%, at least 95%, at least 96%, at least 97%, at least 98%, or at least 99% identity to SEQ ID NO: 4.

SEQ ID NO:4

```
DKPHTCPLCP APELLGGPSV FLFPPKPKDT LMISRTPEVT CVVVDVSHED PEVKFNWYVD      60
GVEVHNAKTK PREEQYNSTY RVVSVLTVLH QDWLNGKEYK CKVSNKALPA PIEKTISKAK     120
GQPREPQVYT LPPSRDELTK NQVSLTCLVK GFYPSDIAVE WESNGQPENN YKATPPVLDS     180
DGSFFLYSKL TVDKSRWQQG NVFSCSVMHE ALHNHYTQKS LSLSPGK                   227
```

Further, an amino acid sequence of the human immunoglobulin constant region fragment is as shown in SEQ ID NO: 4.

Further, the human immunoglobulin constant region fragment includes amino acid modifications at 1, 2, 3, 4, 5, 6, 7, 8, or more positions compared with SEQ ID NO: 4.

Further, the modification is an amino acid substitution, deletion, or insertion.

Further, the substitution includes one or more of P3T, L8P, L14A, L15E, G17A, A110S, P111S, and A173T.

Further, the insertion is insertion of 1, 2, 3, 4, 5, 6, 7, 8, or more amino acids at an N-terminus of the human immunoglobulin constant region fragment.

Still further, the insertion is insertion of 5 amino acids at an N-terminus of the human immunoglobulin constant region fragment.

Still further, the insertion is insertion of 5 amino acids "EPKSS" at an N-terminus of the human immunoglobulin constant region fragment.

Further, the human immunoglobulin constant region fragment includes an amino acid sequence of SEQ ID NO: 5.

SEQ ID NO:5

```
DKTHTCPPCP  APEAEGAPSV  FLFPPKPKDT  LMISRTPEVT  CVVVDVSHED  PEVKFNWYVD        60
GVEVHNAKTK  PREEQYNSTY  RVVSVLTVLH  QDWLNGKEYK  CKVSNKALPS  SIEKTISKAK        120
GQPREPQVYT  LPPSRDELTK  NQVSLTCLVK  GFYPSDIAVE  WESNGQPENN  YKTTPPVLDS        180
DGSFFLYSKL  TVDKSRWQQG  NVFSCSVMHE  ALHNHYTQKS  LSLSPGK                      227
```

Further, the human immunoglobulin constant region fragment includes an amino acid sequence of SEQ ID NO: 6.

SEQ ID NO:6

```
EPKSSDKTHT  CPPCPAPEAE  GAPSVFLFPP  KPKDTLMISR  TPEVTCVVVD  VSHEDPEVKF        60
NWYVDGVEVH  NAKTKPREEQ  YNSTYRVVSV  LTVLHQDWLN  GKEYKCKVSN  KALPAPIEKT        120
ISKAKGQPRE  PQVYTLPPSR  DELTKNQVSL  TCLVKGFYPS  DIAVEWESNG  QPENNYKTTP        180
PVLDSDGSFF  LYSKLTVDKS  RWQQGNVFSC  SVMHEALHNH  YTQKSLSLSP  G                231
```

Further, the TACI-Fc fusion protein has an amino acid sequence having at least 75%, at least 80%, at least 85%, at least 90%, at least 95%, or at least 99% identity to SEQ ID NO: 7.

SEQ ID NO:7

```
SRVDQEERFP  QGLWTGVAMR  SCPEEQYWDP  LLGTCMSCKT  ICNHQSQRTC  AAFCRSLSCR        60
KEQGKFYDHL  LRDCISCASI  CGQHPKQCAY  FCENKLRSPV  NLPPELDKTH  TCPPCPAPEA        120
EGAPSVFLFP  PKPKDTLMIS  RTPEVTCVVV  DVSHEDPEVK  FNWYVDGVEV  HNAKTKPREE        180
QYNSTYRVVS  VLTVLHQDWL  NGKEYKCKVS  NKALPSSIEK  TISKAKGQPR  EPQVYTLPPS        240
RDELTKNQVS  LTCLVKGFYP  SDIAVEWESN  GQPENNYKTT  PPVLDSDGSF  FLYSKLTVDK        300
SRWQQGNVFS  CSVMHEALHN  HYTQKSLSLS  PGK                                      333
```

Further, the TACI-Fc fusion protein has an amino acid sequence shown in SEQ ID NO: 7.
Further, the TACI-Fc fusion protein has an amino acid sequence shown in SEQ ID NO: 8.

SEQ ID NO:8

```
AMRSCPEEQY  WDPLLGTCMS  CKTICNHQSQ  RTCAAFCRSL  SCRKEQGKFY  DHLLRDCISC        60
ASICGQHPKQ  CAYFCENKLR  SEPKSSDKTH  TCPPCPAPEA  EGAPSVFLFP  PKPKDTLMIS        120
RTPEVTCVVV  DVSHEDPEVK  FNWYVDGVEV  HNAKTKPREE  QYNSTYRVVS  VLTVLHQDWL        180
NGKEYKCKVS  NKALPSSIEK  TISKAKGQPR  EPQVYTLPPS  RDELTKNQVS  LTCLVKGFYP        240
SDIAVEWESN  GQPENNYKTT  PPVLDSDGSF  FLYSKLTVDK  SRWQQGNVFS  CSVMHEALHN        300
HYTQKSLSLS  PGK                                                              313
```

Further, the TACI-Fc fusion protein has an amino acid sequence shown in SEQ ID NO: 9.

SEQ ID NO:9

```
SLSCRKEQGE  YYDHLLRDCI  SCASICGQHP  KQCADFCENK  LRSGSGGGGS  EPKSSDKTHT        60
CPPCPAPEAE  GAPSVFLFPP  KPKDTLMISR  TPEVTCVVVD  VSHEDPEVKF  NWYVDGVEVH        120
NAKTKPREEQ  YNSTYRVVSV  LTVLHQDWLN  GKEYKCKVSN  KALPAPIEKT  ISKAKGQPRE        180
PQVYTLPPSR  DELTKNQVSL  TCLVKGFYPS  DIAVEWESNG  QPENNYKTTP  PVLDSDGSFF        240
LYSKLTVDKS  RWQQGNVFSC  SVMHEALHNH  YTQKSLSLSP  G                            281
```

Further, the drug targeting BLyS and/or APRIL is telitacicept, atacicept, or povetacicept.

Further, the TACI-Fc fusion protein is telitacicept.

Further, the ANCA-associated vasculitis includes, but is not limited to, one or more of granulomatosis with polyangiitis (GPA), eosinophilic granulomatosis with polyangiitis (EGPA), and microscopic polyangiitis (MPA).

In some preferred embodiments, the ANCA-associated vasculitis is granulomatosis with polyangiitis; in other preferred embodiments, the ANCA-associated vasculitis is eosinophilic granulomatosis with polyangiitis; in other preferred embodiments, the ANCA-associated vasculitis is microscopic polyangiitis; in other preferred embodiments, the ANCA-associated vasculitis is granulomatosis with polyangiitis comorbid with eosinophilic granulomatosis with polyangiitis; in other preferred embodiments, the ANCA-associated vasculitis is granulomatosis with polyangiitis comorbid with microscopic polyangiitis; in other preferred embodiments, the ANCA-associated vasculitis is eosinophilic granulomatosis with polyangiitis comorbid with microscopic polyangiitis; and in other preferred embodiments, the ANCA-associated vasculitis is granulomatosis with polyangiitis comorbid with eosinophilic granulomatosis with polyangiitis comorbid with microscopic polyangiitis. It can be understood that clinically, a disease type of ANCA-associated vasculitis may be classified (i.e., further determined as granulomatosis with polyangiitis, eosinophilic granulomatosis with polyangiitis, microscopic polyangiitis, or a combination of two or three), or may not be classified (i.e., collectively referred to as ANCA-associated vasculitis); therefore, the disease type should not be regarded as a limitation of the disease.

Further, the method includes administering a therapeutically effective amount of a drug targeting BLyS and/or APRIL to a patient having the ANCA-associated vasculitis during an induction of remission treatment phase and/or a maintenance of remission treatment phase thereof (the drug targeting BLyS and/or APRIL is further preferably a TACI-Fc fusion protein, and still more preferably telitacicept).

In some preferred embodiments, the method includes administering a therapeutically effective amount of a drug targeting BLyS and/or APRIL to a patient having the ANCA-associated vasculitis during an induction of remission treatment phase thereof. In other preferred embodiments, the method includes administering a therapeutically effective amount of a drug targeting BLyS and/or APRIL to a patient having the ANCA-associated vasculitis during a maintenance of remission treatment phase thereof. In other preferred embodiments, the method includes administering a therapeutically effective amount of a drug targeting BLyS and/or APRIL to a patient having the ANCA-associated vasculitis during both an induction of remission treatment phase and a maintenance of remission treatment phase thereof. The drug targeting BLyS and/or APRIL is further preferably a TACI-Fc fusion protein, and still more preferably telitacicept.

Further, the patient is an adult patient or a pediatric patient. In some preferred embodiments, the patient is an adult patient. In other preferred embodiments, the patient is a pediatric patient.

Further, the patient with the ANCA-associated vasculitis is in a newly diagnosed disease treatment phase or a recurrent disease treatment phase. In some preferred embodiments, the patient with ANCA-associated vasculitis is in a newly diagnosed disease treatment phase (i.e., the patient is first diagnosed with ANCA-associated vasculitis). In other preferred embodiments, the patient with ANCA-associated vasculitis is in a recurrent disease treatment phase. That is, the drug targeting BLyS and/or APRIL provided by the present invention (further preferably a TACI-Fc fusion protein, and still more preferably telitacicept) can be applied both in the newly diagnosed disease treatment phase of the patient and in the recurrent disease treatment phase of the patient.

Further, the patient with the ANCA-associated vasculitis is an inactive-phase patient, an active-phase severe patient, or an active-phase non-severe patient. In some preferred embodiments, the patient with ANCA-associated vasculitis is an inactive-phase patient. In other preferred embodiments, the patient is an active-phase severe patient. In other preferred embodiments, the patient is an active-phase non-severe patient.

Further, the patient with the ANCA-associated vasculitis is comorbid with one or more other autoimmune diseases. The autoimmune diseases (referring to the list of diseases listed by the American Autoimmune Related Diseases Association) include, but are not limited to: psoriasis, Hashimoto's thyroiditis, systemic lupus erythematosus, rheumatoid arthritis, type 1 diabetes mellitus, toxic diffuse goiter, inflammatory bowel disease, celiac disease, achalasia, Addison's disease, adult-onset Still's disease, agammaglobulinemia, alopecia areata, amyloidosis, ankylosing spondylitis, anti-glomerular basement membrane antibody nephritis, antiphospholipid syndrome, autoimmune angioedema, autoimmune autonomic ganglionopathy, autoimmune encephalomyelitis, autoimmune hepatitis, autoimmune inner ear disease, autoimmune myocarditis, autoimmune oophoritis, autoimmune orchitis, autoimmune pancreatitis, autoimmune retinopathy, autoimmune urticaria, acute motor-sensory axonal neuropathy, Balo disease, Behcet's disease, benign mucosal pemphigoid, bullous pemphigoid, Castleman disease, celiac disease, Chagas disease, chronic inflammatory demyelinating polyneuropathy, chronic recurrent multifocal osteomyelitis, Churg-Strauss syndrome, Cogan syndrome, cold agglutinin disease, congenital heart block, coxsackievirus myocarditis, CREST syndrome, Crohn's disease, dermatitis herpetiformis, dermatomyositis, Devic's disease, discoid lupus, Dressler syndrome, endometriosis, eosinophilic esophagitis, eosinophilic fasciitis, erythema nodosum, essential mixed cryoglobulinemia, Evans syndrome, fibromyalgia, fibrosing alveolitis, giant cell arteritis, giant cell myocarditis, glomerulonephritis, Goodpasture syndrome, granulomatosis with polyangiitis, Graves disease, Guillain-Barre syndrome, Hashimoto's thyroiditis, hemolytic anemia, Henoch-Schonlein purpura, herpes gestationis or gestational pemphigoid, hidradenitis suppurativa, hypogammaglobulinemia, IgA nephropathy, IgG4-related sclerosing disease, immune thrombocytopenic purpura, inclusion body myositis, interstitial cystitis, juvenile arthritis, juvenile myositis, Kawasaki disease, Lambert-Eaton syndrome, leukocytoclastic vasculitis, lichen planus, lichen sclerosus et atrophicus, ligneous conjunctivitis, linear IgA disease, chronic Lyme disease, Meniere's disease, microscopic polyangiitis, mixed connective tissue disease, Mooren's ulcer, Mucha-Habermann disease, multifocal motor neuropathy, multiple sclerosis, myasthenia gravis, myositis, narcolepsy, neonatal lupus, neutropenia, ocular cicatricial pemphigoid, optic neuritis, palindromic rheumatism, PANDAS, paraneoplastic cerebellar degeneration, paroxysmal nocturnal hemoglobinuria, Parry-Romberg syndrome, pars planitis, Parsonage-Turner syndrome, pemphigus, peripheral neuropathy, perivenous encephalomyelitis, pernicious anemia, POEMS syndrome, polyarteritis nodosa, polyglandular syndromes type I, II, III, polymyalgia rheumatica, polymyositis, postmyocardial infarction syndrome, postpericardiotomy syndrome, primary biliary cirrhosis, primary sclerosing cholangitis, progesterone dermatitis, psoriatic arthritis, pure red cell aplasia, pyoderma gangrenosum, Raynaud's phenomenon, reactive arthritis, reflex sympathetic dystrophy, relapsing polychondritis, restless legs syndrome, retroperitoneal fibrosis, rheumatic fever, sarcoidosis, Schmidt syndrome, scleritis, scleroderma, Sjögren's syndrome, sperm and testicular autoimmunity, stiff person syndrome, subacute bacterial endocarditis, Susac syndrome, sympathetic ophthalmia, Takayasu arteritis, temporal arteritis, thyroid eye disease, Tolosa-Hunt syndrome, ulcerative colitis, undifferentiated connective tissue disease, uveitis, vasculitis, vitiligo, Vogt-Koyanagi-Harada disease, and antibody-type autoimmune hemolytic anemia.

Further, the patient with the ANCA-associated vasculitis has involvement of one or more of the lungs, and/or kidneys, and/or gastrointestinal tract, and/or nervous system, and/or eyes, ears, nose, and throat, and/or systemic musculoskeletal system, and/or serous membrane, and/or blood system, and/or other tissues or organs.

Further, the patient has not previously received a treatment regimen for ANCA-associated vasculitis.

Further, the patient has previously received a treatment regimen for ANCA-associated vasculitis.

Further, the treatment regimen for ANCA-associated vasculitis includes, but is not limited to, one or more of a glucocorticoid treatment regimen, an immunosuppressant treatment regimen, a biological agent treatment regimen, and plasma exchange.

In some preferred embodiments, the patient has previously received a treatment regimen for ANCA-associated vasculitis, and the treatment regimen for ANCA-associated vasculitis is a glucocorticoid treatment regimen, an immunosuppressant treatment regimen, a biological agent treatment regimen, plasma exchange, a combined application regimen of glucocorticoids + immunosuppressants, a combined application regimen of glucocorticoids + biological agents, a combined application regimen of glucocorticoids + plasma exchange, a combined application regimen of immunosuppressants + biological agents, a combined application regimen of immunosuppressants + plasma exchange, a combined application regimen of biological agents + plasma exchange, a combined application regimen of glucocorticoids + immunosuppressants + biological agents, a combined application regimen of glucocorticoids + immunosuppressants + plasma exchange, a combined application regimen of immunosuppressants + biological agents + plasma exchange, or a combined application regimen of glucocorticoids + immunosuppressants + biological agents + plasma exchange.

Further, the method includes administering a therapeutically effective amount of a drug targeting BLyS and/or APRIL to a patient having the ANCA-associated vasculitis, while one or more of a glucocorticoid, an immunosuppressant, a biological agent, and plasma exchange are applied in combination.

In some preferred embodiments, the method includes administering a therapeutically effective amount of a drug targeting BLyS and/or APRIL to a patient having the ANCA-associated vasculitis. In other preferred embodiments, the method includes administering a drug targeting BLyS and/or APRIL, a glucocorticoid, and an immunosuppressant in combination; or administering a drug targeting BLyS and/or APRIL, a glucocorticoid, and a biological agent in combination; or administering a drug targeting BLyS and/or APRIL, a glucocorticoid, and plasma exchange in combination; or administering a drug targeting BLyS and/or APRIL, an immunosuppressant, and a biological agent in combination; or administering a drug targeting BLyS and/or APRIL, an immunosuppressant, and plasma exchange in combination; or administering a drug targeting BLyS and/or APRIL, a biological agent, and plasma exchange in combination; or administering a drug targeting BLyS and/or APRIL, a glucocorticoid, an immunosuppressant, and a biological agent in combination; or administering a drug targeting BLyS and/or APRIL, a glucocorticoid, an immunosuppressant, and plasma exchange in combination; or administering a drug targeting BLyS and/or APRIL, an immunosuppressant, a biological agent, and plasma exchange in combination to a patient having the ANCA-associated vasculitis. Preferably, the combined administration includes simultaneous application or phased application.

Preferably, the biological agents described in the present invention include, but are not limited to: rituximab, mepolizumab, avacopan, belimumab, etanercept, benralizumab, depemokimab, abatacept, obinutuzumab, alemtuzumab, tocilizumab, and vilobelimab.

Further, a single administration dose of the drug targeting BLyS and/or APRIL is about 0.1 to 10 mg/kg, and further includes 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, and 10 mg/kg.

Further, a single administration dose of the drug targeting BLyS and/or APRIL is 40-240 mg, and further preferably includes 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, and 240 mg.

Further, a method for detecting a protein content of the above drug is: ultraviolet-visible spectrophotometry, which determines an absorbance value of a telitacicept sample at 280 nm according to the maximum ultraviolet absorption of proteins at the wavelength. After correcting the absorbance at 320 nm, the obtained absorbance value at 280 nm is proportional to a protein concentration. The protein concentration is calculated according to the Lambert-Beer law to determine the protein content. The calculation formula for protein content is as follows:

$$\text{Proteins content (mg/ml)} = \frac{A280 \text{ (or } A280 \text{ (corrected))}}{\varepsilon} \times \text{sample dilution factor}$$

In the formula: $\varepsilon$ is an extinction coefficient value of telitacicept, with a unit of $(mg/ml)^{-1} \cdot cm^{-1}$;

$A_{280}$ is an average absorbance value of a sample solution at 280 nm; and

$A_{280}$ (corrected) is an average absorbance value of the sample solution at 280 nm after correction.

Further, the drug targeting BLyS and/or APRIL is used 1-8 times during each one-month interval, i.e., an administration frequency of the TACI-Fc fusion protein is 1, 2, 3, 4, 5, 6, 7, or 8 times a month.

Further, the drug targeting BLyS and/or APRIL is used 1-8 times during each two-month interval, i.e., an administration frequency of the TACI-Fc fusion protein is 1, 2, 3, 4, 5, 6, 7, or 8 times every two months.

Further, the drug targeting BLyS and/or APRIL is used 1-8 times during each three-month interval, i.e., an administration frequency of the TACI-Fc fusion protein is 1, 2, 3, 4, 5, 6, 7, or 8 times every three months.

Further, an administration frequency of the drug targeting BLyS and/or APRIL is once a week, twice a week, or three times a week.

Further, an administration frequency of the drug targeting BLyS and/or APRIL is once every two weeks, once every three weeks, or once a month.

Further, an administration frequency of the drug targeting BLyS and/or APRIL is on-demand administration.

Further, administration of the drug targeting BLyS and/or APRIL includes continuous administration and/or intermittent administration.

Further, administration of the drug targeting BLyS and/or APRIL includes administration at regular intervals.

Further, administration of the drug targeting BLyS and/or APRIL includes administration at irregular intervals.

Further, an administration mode of the drug targeting BLyS and/or APRIL includes subcutaneous, intramuscular, or intravenous administration, and an administration site is preferably the thigh, abdomen, or upper arm. In some specific embodiments, an administration mode of the TACI-Fc fusion protein includes subcutaneous injection, intramuscular injection, or intravenous injection.

Further, an injection site for each injection of the drug targeting BLyS and/or APRIL is the same or different. In some specific embodiments, an injection site for each injection of the TACI-Fc fusion protein is the same; and in other specific embodiments, an injection site for each injection of the TACI-Fc fusion protein is different.

The present invention also provides a method for treating a patient with ANCA-associated vasculitis who has previously received a treatment regimen for ANCA-associated vasculitis, said method includes (1) determining whether the patient has previously received a treatment regimen for ANCA-associated vasculitis, and (2) if the patient has previously received treatment for ANCA-associated vasculitis, administering an effective amount of a drug targeting BLyS and/or APRIL, further being a TACI-Fc fusion protein, to the patient with the ANCA-associated vasculitis.

The drug targeting BLyS and/or APRIL provided by the present invention exhibits unexpected clinical efficacy and good safety in the treatment of ANCA-associated vasculitis. Specifically, the application of the drug targeting BLyS and/or APRIL provided by the present invention (taking telitacicept as an example) in patients with ANCA-associated vasculitis exhibits better safety and superior therapeutic effects compared with existing treatment regimens/means. Specifically, for patients with ANCA-associated vasculitis, the drug targeting BLyS and/or APRIL provided by the present invention (taking telitacicept as an example) shows a good therapeutic prospect both in the induction phase and the maintenance phase; moreover, even after patients have undergone induction of remission, the subsequent application of the drug targeting BLyS and/or APRIL provided by the present invention (taking telitacicept as an example) can further improve the patient's condition, presenting a considerable effect. For patients who have failed treatment with glucocorticoids, and/or glucocorticoids + immunosuppressants, and/or glucocorticoids + immunosuppressants + biological agents (such as rituximab), or who have relapsed repeatedly after treatment, the drug targeting BLyS and/or APRIL provided by the present invention (taking telitacicept as an example) also shows a good therapeutic prospect. For patients with ANCA-associated vasculitis comorbid with one or more other autoimmune diseases (including, but not limited to, Sjögren's syndrome, systemic lupus erythematosus, nephrotic syndrome, and lupus nephritis), the drug targeting BLyS and/or APRIL provided by the present invention (taking telitacicept as an example) also shows a good therapeutic prospect. The drug targeting BLyS and/or APRIL provided by the present invention (taking telitacicept as an example) exhibits better safety and superior therapeutic effects relative to glucocorticoids and/or immunosuppressants, especially in patients with severe ANCA-associated vasculitis. For patients with a long medical history and repeated relapses after treatment, the drug targeting BLyS and/or APRIL provided by the present invention (taking telitacicept as an example) shows good therapeutic potential. For patients with severe ANCA-associated vasculitis and severe renal damage, the drug targeting BLyS and/or APRIL provided by the present invention (taking telitacicept as an example) exhibits good therapeutic effects while also showing good safety.

## BRIEF DESCRIPTION OF THE DRAWINGS

FIG. 1 shows treatment guidelines for granulomatosis with polyangiitis (GPA) and microscopic polyangiitis (MPA).

FIG. 2 shows treatment guidelines for eosinophilic granulomatosis with polyangiitis (EGPA).

FIG. 3 shows changes in serum creatinine levels of a patient in Case 1 of Example 1.

## DETAILED DESCRIPTION

Unless defined otherwise, all technical and scientific terms used herein have the same meaning as commonly understood by one of ordinary skill in the art to which this invention belongs. Regarding definitions and terminology in the art, professionals can specifically refer to Current Protocols in Molecular Biology (Ausubel).

The three-letter codes and one-letter codes for amino acids used in the present invention are as described in J. Biol. Chem., 243, p. 3558 (1968).

The term "TACI" in the present invention refers to transmembrane activator and CAML interactor, which is a member of the tumor necrosis factor receptor superfamily. The term "BLyS" in the present invention refers to B lymphocyte stimulator, which is a member of the TNF ligand superfamily existing in 2 forms: membrane-bound and soluble. It is specifically expressed on the surface of bone marrow cells and selectively stimulates B lymphocyte proliferation and the production of immunoglobulins. The term "APRIL" (a proliferation-inducing ligand) in the present invention refers to a tumor necrosis factor (TNF) analog that can stimulate the proliferation of naive B cells and T cells in the body, promote B cell accumulation, and increase spleen content. APRIL can specifically bind to TACI and BCMA; upon binding, it can prevent APRIL from binding to B cells and inhibit the proliferation response of naive B cells stimulated by APRIL. Furthermore, APRIL can competitively bind to receptors (BCMA, TACI) with BLyS.

The term "TACI-Fc fusion protein" referred to in the present invention refers to a transmembrane activator and calcium modulator and cyclophilin ligand interactor (TACI)-immunoglobulin fusion protein (i.e., TACI-Fc fusion protein). The TACI-immunoglobulin fusion protein provided by the present invention includes: (i) an extracellular domain of TACI or a fragment thereof binding BLyS and/or APRIL; and (ii) a human immunoglobulin constant region fragment.

The term "extracellular domain of TACI or fragment thereof binding BLyS and/or APRIL" in the present invention includes the extracellular domains of TACI disclosed in U.S. Patent Nos. 5,969,102, 6,316,222, and 6,500,428, and U.S. Patent Applications 09/569,245 and 09/627,206 (the contents of which are incorporated herein by reference), as well as specific fragments of the extracellular domain of TACI that can interact with TACI ligands, and the amino acid fragment at positions 13-118 of the extracellular domain of TACI disclosed in Chinese Patent Publication No. CN101323643A.

Examples of "TACI-Fc fusion proteins" include telitacicept (amino acid sequence: SEQ ID NO: 7), atacicept (amino acid sequence: SEQ ID NO: 8), and povetacicept (amino acid sequence: SEQ ID NO: 9).

In the term "human immunoglobulin constant region fragment" referred to in the present invention, the immunoglobulin portion is preferably IgG1, which may include a heavy chain constant region, such as a human heavy chain constant region. The preferred "human immunoglobulin constant region fragment" of the present invention is an amino acid fragment containing a partial hinge region domain, a CH2 domain, and a CH3 domain. In some more preferred embodiments, the amino acid sequence of the "human immunoglobulin constant region fragment" described in the present invention is as shown in SEQ ID NO: 4, or includes an amino acid sequence having at least 90%, at least 91%, at least 92%, at least 93%, at least 94%, at least 95%, at least 96%, at least 97%, at least 98%, or at least 99% identity to SEQ ID NO: 4. In some more preferred embodiments, the amino acid sequence of the "human immunoglobulin constant region fragment" is as shown in SEQ ID NO: 5.

The term "treatment" referred to in the present invention, in relation to a given disease or condition, includes, but is not limited to: inhibiting the disease or condition, e.g., arresting the development of the disease or condition; alleviating the disease or condition, e.g., causing regression of the disease or condition; or alleviating symptoms caused by the disease or condition, e.g., alleviating, preventing, or treating symptoms of the disease or condition; or reducing the chance of disease recurrence or preventing disease recurrence.

The term "remission" as referred to in the present invention refers to the absence of clinical symptoms or signs related to GPA, MPA, or EGPA at the start or end of treatment.

The term "amino acid" referred to in the present invention is understood in the broadest sense, referring generally to a class of organic compounds containing amino and carboxyl groups. Preferably, the amino acids involved in the present invention are the main units composing proteins in living organisms, including but not limited to: glycine, alanine, valine, leucine, isoleucine, methionine, proline, tryptophan, serine, tyrosine, cysteine, phenylalanine, asparagine, glutamine, threonine, aspartic acid, glutamic acid, lysine, arginine, and histidine.

The three-letter codes and one-letter codes for amino acids used in the present invention are as described in J. Biol. Chem., 243, p. 3558 (1968). There are various numbering systems for amino acid positions, such as the Kabat numbering system, EU numbering system, sequential numbering, etc. In the present invention, the "sequential numbering" system is adopted for amino acid positions. For example, "positions 3, 8, 14, 15, 17, 110, 111, or 173 of SEQ ID NO: 4" as described in the present invention refers to the 3rd amino acid, 8th amino acid of SEQ ID NO: 4, and so on; "P3T" as described in the present invention refers to mutating the amino acid at position 3 of SEQ ID NO: 4 from the original "P" to "T", and "L8P" refers to mutating the amino acid at position 8 of SEQ ID NO: 4 from the original "L" to "P", and so forth.

As an optional implementation, the constant region of the immunoglobulin provided by the present invention may introduce one or more amino acid changes, such as substitution (i.e., mutation), addition (i.e., insertion), or deletion (i.e., deletion).

The term "telitacicept" (also referred to as "Tai'ai", which are used interchangeably in the present invention) in the present invention is a TACI-Fc fusion protein with the INN name telitacicept, whose amino acid sequence is as shown in SEQ ID NO: 7, or refer to https://extranet.who.int/soinn/mod/page/view.php?id=137&inn_n=10932.

The TACI-Fc fusion protein of the present invention can be administered via any of a variety of routes, including but not limited to: oral, intravenous injection, intramuscular injection, intra-arterial injection, intramedullary injection, intraperitoneal injection, intrathecal injection, intracerebral, transdermal, transcutaneous, topical, subcutaneous, intranasal, enteral, sublingual, intravaginal, or rectal routes, etc.

The term "ANCA-associated vasculitis" in the present invention, i.e., antineutrophil cytoplasmic antibody (ANCA)-associated vasculitis (AAV), is a vasculitis characterized by necrotizing inflammation, including microscopic polyangiitis (MPA), granulomatosis with polyangiitis (GPA), and eosinophilic granulomatosis with polyangiitis (EGPA).

The term "microscopic polyangiitis" in the present invention, also known as microscopic polyarteritis, is a systemic necrotizing vasculitis without immunoglobulin deposition (pauci-immune deposition), mainly involving small blood vessels including veins. The disease may present as pulmonary-renal syndrome, manifesting as rapidly progressive glomerulonephritis and alveolar hemorrhage, but the disease type depends on the involved organs.

The term "granulomatosis with polyangiitis" in the present invention, previously known as Wegener's granulomatosis (WG), is a necrotizing granulomatous vasculitis and an autoimmune disease. The lesions of this disease involve small arteries, veins, and capillaries, and occasionally large arteries. Its pathology is characterized by inflammation of the vascular wall, mainly invading the upper and lower respiratory tracts and kidneys. It usually starts with focal granulomatous inflammation of the nasal mucosa and lung tissue, and then progresses to diffuse necrotizing granulomatous inflammation of blood vessels. Clinically, it often presents as rhinitis and sinusitis, lung lesions, and progressive renal failure. It may also involve joints, eyes, and skin, and may further invade the eyes, heart, nervous system, ears, and the like.

The term "eosinophilic granulomatosis with polyangiitis" in the present invention, also known as Churg-Strauss syndrome (CSS), is a primary systemic autoimmune vasculitis characterized by the pathological triad of tissue eosinophilia, granulomatous inflammation, and vasculitis. This type of vasculitis is often accompanied by asthma and eosinophilia. Patients may have perinuclear antineutrophil cytoplasmic antibodies against myeloperoxidase. Such autoantibodies are more common in patients with prominent vasculitis symptoms, such as those with glomerulonephritis.

The term "biological agent treatment regimen" in the present invention is commonly used for the treatment of patients with traditional treatment failure, glucocorticoid resistance or intolerance, relapse during glucocorticoid tapering, refractory disease, or severe disease. Exemplary promising biological targeted therapies currently include:

(1) B cell depletion therapy: such as, but not limited to, anti-CD20 monoclonal antibodies, anti-CD19 monoclonal antibodies, B lymphocyte activating factor (BAFF) inhibitors, etc.;

(2) targeting T lymphocytes: such as, but not limited to, abatacept, signaling lymphocytic activation molecule family member 7 (SLAMF7) monoclonal antibodies, inducible costimulator ligand (ICOSL) inhibitors, etc.; and

(3) cytokine-targeted therapies (targeting IL-4, IL-5, TNF-α) and Janus kinase (JAK) inhibitors targeting intracellular signaling pathways, etc.

The term "inactive phase/active phase" described in the present invention refers to the presence of new, persistent, or worsening clinical symptoms and/or signs related to GPA, MPA, or EGPA.

The term "glucocorticoid" described in the present invention refers to a regulatory molecule that plays an important regulatory role in the body's development, growth, metabolism, and immune function. It is the most important regulatory hormone for the body's stress response, and also the most widely used and effective anti-inflammatory agent in clinical practice. Preferably, the glucocorticoids involved in the present invention include, but are not limited to: prednisone, prednisolone, methylprednisolone, betamethasone, beclomethasone dipropionate, hydrocortisone, dexamethasone, etc.

The term "immunosuppressant" described in the present invention refers to a drug that inhibits the body's immune response. It can inhibit the proliferation and function of cells involved in the immune response (such as macrophages including T cells and B cells), and is also known as conventional synthetic disease-modifying anti-rheumatic drugs (cDMARDs). Preferably, the immunosuppressants described in the present invention include, but are not limited to: mycophenolate mofetil, azathioprine, methotrexate, leflunomide, cyclophosphamide, cyclosporine, tacrolimus, iguratimod, thalidomide, and other drugs that inhibit the body's immune response.

The term "severe disease" described in the present invention refers to the presence of life-threatening symptoms or organ manifestations (such as alveolar hemorrhage, glomerulonephritis, central nervous system vasculitis, etc.).

The term "about" in the present invention is used to indicate that a value includes inherent error variations of the device or method used to determine the value, or variations existing between the tested samples. Unless otherwise stated or clearly apparent from the context, the term "about" means within 10% above or below the reported value (unless such a number would exceed 100% of the possible value or fall below 0%). When used in conjunction with a numerical range or series, the term "about" applies to the endpoints of the range or each numerical value listed in the series, unless otherwise stated.

The implementations of the present invention will be described in detail below with reference to examples, but those skilled in the art will understand that the following examples are only used to illustrate the present invention and should not be regarded as limiting the scope of the present invention.

Example 1 Therapeutic Effects in Real-World Cases

[Case 1]

A patient experienced sudden abdominal pain, cough with sputum, and intermittent hemoptysis without an obvious cause. Clinically, the patient was diagnosed with ANCA-associated vasculitis (comorbid with lung and kidney injury) based on multiple indicators. The patient had a history of multiple episodes of aortic intramural hematoma and could not tolerate cyclophosphamide pulse therapy due to lung lesions, lymphocyte count, and other factors.

After treatment with glucocorticoids (sodium methylprednisolone succinate for injection, 250 mg) + immunosuppressants (cyclophosphamide, 0.4 g) + telitacicept (160 mg subcutaneous injection, once a week), the cough and sputum improved significantly, and there was no hemoptysis. Subsequently, the patient received renal replacement therapy and underwent peritoneal dialysis catheterization. Subsequently, the patient continued to receive treatment with the glucocorticoid prednisone (25 mg/day) + intermittent telitacicept treatment + peritoneal dialysis treatment + treatment for chronic kidney disease complications, and most indicators and signs recovered.

This patient was treated with glucocorticoids, low-dose cyclophosphamide combined with telitacicept, and the clinical symptom of hemoptysis improved significantly. As shown in FIG. 3, the serum creatinine level tended to be stable after treatment, and renal function was relatively stable. Results showed:

① For patients with severe ANCA-associated vasculitis, telitacicept is safer relative to cyclophosphamide and can alleviate pulmonary symptoms; that is, telitacicept exhibits better safety compared with cyclophosphamide.

② For patients intolerant to cyclophosphamide, telitacicept exhibits better therapeutic effects.

[Case 2]

A patient had a 10-year medical history and was further diagnosed with microscopic polyangiitis (involving the lungs and kidneys) comorbid with Sjögren's syndrome based on multiple indicators.

The patient had previously relapsed after achieving remission with glucocorticoid (methylprednisolone injection, prednisone) treatment. Subsequently, the patient was treated with glucocorticoids (methylprednisolone) + immunosuppressants (cyclophosphamide), but the symptoms were not relieved, and fever, and cough with sputum still occurred. In the later stage, re-examination showed that the inflammation indicators rose again, and MPA was not controlled. Subsequently, the treatment regimen was adjusted to "glucocorticoids (methylprednisolone powder for injection) + immunosuppressants (cyclophosphamide) + telitacicept (80 mg, once a week)". After treatment, the patient's clinical symptoms of fever, cough with sputum, and fatigue were relieved, lung symptoms were alleviated, renal function was relatively stable, myeloperoxidase (MPO) antibodies decreased, ferritin decreased, and the erythrocyte sedimentation rate (ESR) decreased.

After treatment with glucocorticoids, low-dose cyclophosphamide combined with telitacicept, the patient's lung symptoms were alleviated, renal function was relatively stable, and pANCA antibody levels and abnormal B cells gradually returned to normal. Results showed:

① For patients with microscopic polyangiitis (MPA), the effect of telitacicept induction therapy is excellent;

② Compared with the glucocorticoid and the glucocorticoid + immunosuppressant treatment regimens, the glucocorticoid + immunosuppressant + telitacicept treatment regimen has a better therapeutic effect.

③ For patients with ANCA-associated vasculitis comorbid with other autoimmune diseases (such as Sjögren's syndrome), telitacicept shows good therapeutic potential.

④ For patients with a long medical history and recurrent disease after treatment, telitacicept exhibits good therapeutic potential.

[Case 3]

A patient was clinically diagnosed with ANCA-associated vasculitis and ANCA-associated glomerulonephritis (accompanied by extremely high-risk hypertension, interstitial pneumonia, and pulmonary infection) based on multiple indicators for the first time.

During the induction of remission treatment phase, hormone + CTX treatment was adopted: methylprednisolone 500 mg pulse therapy was administered for 3 days, followed by tapering to 40 mg qd, then 32 mg qd; during the treatment cycle, cyclophosphamide 0.4 g pulse therapy was administered separately, with a cumulative CTX dose of 3.2 g. During the maintenance of remission treatment phase, hormone + telitacicept treatment was adopted: glucocorticoids (methylprednisolone tablets) + telitacicept (160 mg/time, once a week, for 4 consecutive times).

Later, the patient discontinued the drug for 3 months due to COVID-19 infection, followed by a rebound in MPO antibodies. Subsequently, treatment was administered according to glucocorticoids (methylprednisolone tablets) + telitacicept (160 mg/time, once a week, for 2 consecutive times), and MPO antibodies were stably controlled.

Results showed:

① This patient was a newly diagnosed patient with ANCA-associated vasculitis. After standard induction therapy (hormone + cyclophosphamide) and maintenance treatment (hormone + telitacicept), the MPO level could be further reduced, renal function was stabilized, and progression of renal damage was avoided. This indicates that the use of the hormone + telitacicept regimen during the maintenance treatment phase has a considerable effect on reducing the autoantibody level in ANCA patients and avoiding the progression of renal function;

② The patient discontinued the drug for 3 months due to "COVID-19 infection" during maintenance treatment, and the MPO level rose. The addition of telitacicept reduced the MPO level. This indicates that the use of hormone + telitacicept in recurrent disease patients is conducive to limiting ANCA activity and is expected to achieve the goal of autoantibody seroconversion to negative.

[Case 4]

A patient was clinically diagnosed with ANCA-associated vasculitis complicated with renal damage based on multiple indicators. In the early stage, after seven years of standard diagnosis and treatment with "glucocorticoids (prednisone) + immunosuppressants (CellCept, i.e., mycophenolate mofetil)", the effect was unsatisfactory, and ANCA remained persistently positive.

Subsequently, the patient received treatment with glucocorticoids (prednisone) + immunosuppressants (CellCept) + rituximab (MabThera, 100 mg/time). Although B-cell depletion was achieved after 1 year, ANCA never turned negative, and a downward trend in renal function began to appear, with persistent proteinuria.

Subsequently, the patient was treated with hormones (methylprednisolone) + immunosuppressants (cyclophosphamide) + telitacicept, supplemented with anti-infection, blood pressure lowering, kidney protection, calcium supplementation, stomach protection, and other symptomatic treatments. The patient experienced no obvious discomfort after using hormones and cyclophosphamide. During the entire treatment period, telitacicept was administered 9 times consecutively at intervals (160 mg/time), with varying intervals between each administration. During this period, CD19+B cells remained at a low level, MPO remained stable, and renal function remained relatively stable. Six months after drug withdrawal, CD19+B cells recovered to normal levels, MPO increased significantly, and renal function decreased; subsequently, after using telitacicept again once, CD19+B cells decreased, MPO decreased significantly, and renal function was improved. The relevant test data are shown in Table 1:

Table 1. Changes in patient MPO and renal function

| Time | MPO (RU/mL) | Creatinine ($\mu$mol/L) | eGFR (mL/min) | Dosing status |
|---|---|---|---|---|
| 0 | 49.55 | 74 | 93.31 | - |
| 3 m | 49.01 | 78 | 87.42 | Dosing |
| 4 m | 46.99 | - | - | Dosing |
| 12 m | 78.07 | 81 | 83.1 | Drug withdrawal |
| 16 m | 39.19 | 69 | 100.74 | Dosing |

Results showed:

① For patients with a long medical history and poor response to multiple treatment regimens, telitacicept exhibits good therapeutic potential.

② For patients with unsatisfactory response to hormone + immunosuppressant treatment (e.g., ANCA never turned negative), telitacicept exhibits good therapeutic potential.

③ For patients whose ANCA remained positive after rituximab treatment, telitacicept exhibits good therapeutic potential.

④ For patients with decreased renal function after rituximab treatment, telitacicept exhibits better safety.

⑤ For patients with refractory ANCA-associated vasculitis, continuous use of telitacicept yields considerable B-cell depletion effect.

⑥ Continuous use of telitacicept is conducive to limiting ANCA activity and is expected to achieve the goal of autoantibody seroconversion to negative.

⑦ Continuous use of telitacicept is expected to delay the progression of renal function in patients with ANCA-associated glomerulonephritis.

[Case 5]

A patient was admitted to the hospital with fever and proteinuria without an obvious cause. Clinically, the patient was diagnosed with ANCA-positive comorbid with proliferative systemic lupus erythematosus nephritis based on multiple indicators. The patient had a history of recurrence after treatment with glucocorticoids (prednisolone acetate) + immunosuppressants (mycophenolate mofetil dispersible tablets).
Later, after treatment with hormones + MMF and other symptomatic treatments (anti-inflammatory, blood pressure lowering), renal function was basically stable, urine protein was between 500-750 mg/day, and urine red blood cells were 20-25/hpf. Subsequently, the treatment regimen was adjusted to hormone + MMF + telitacicept (initial dose: 160 mg qw, adjusted to 80 mg qw after 5 months of treatment). After treatment, urine red blood cells remained less than 10/hpf, and renal function was stable.
Results showed:

① For patients with ANCA comorbid with other autoimmune diseases (such as proliferative systemic lupus erythematosus nephritis), after the combined use of telitacicept, the patient's urine red blood cells remained less than 10/hpf, and renal function reached a stable state. This indicates that for patients with ANCA comorbid with other autoimmune diseases, telitacicept exhibits good therapeutic potential.

② After experiencing induction of remission, the application of telitacicept can further improve the patient's condition.

[Case 6]

A patient was clinically diagnosed with: ANCA-associated glomerulonephritis (renal injury), comorbid with systemic lupus erythematosus, nephrotic syndrome, lupus nephritis, and acute renal failure, etc., based on multiple indicators.

The first treatment regimen adopted was glucocorticoids + immunosuppressants: methylprednisolone 500 mg × 3d, hydroxychloroquine 200 mg bid + methylprednisolone 40 mg initially, combined with cyclophosphamide pulse therapy. Later, due to a significant increase in the patient's serum creatinine, accompanied by electrolyte and acid-base imbalance, and significant gastrointestinal symptoms in the patient, continuous hemodialysis treatment was administered for 3 days during the treatment period. Subsequently, the dosage regimen was adjusted: glucocorticoids (methylprednisolone tablets, 40 mg qd) + telitacicept (160 mg subcutaneous injection) and other symptomatic regimens (hydroxychloroquine sulfate tablets 200 mg bid), combined with renal replacement therapy (peritoneal dialysis catheterization) and intermittent cyclophosphamide pulse therapy. After treatment, re-examination showed no lupus activity in the patient, and creatinine decreased significantly compared with before. Subsequently, the patient continued to receive glucocorticoids + hydroxychloroquine + intermittent cyclophosphamide + telitacicept treatment.

After treatment with glucocorticoids, hydroxychloroquine, cyclophosphamide combined with telitacicept, the patient's clinical symptoms improved significantly, renal function was relatively stable, and dialysis treatment was discontinued. Results showed:

① For patients with severe ANCA-associated vasculitis comorbid with multiple autoimmune diseases (such as those concurrently comorbid with systemic lupus erythematosus, nephrotic syndrome, and lupus nephritis), telitacicept can still exhibit good therapeutic potential.

② For patients with severe ANCA-associated vasculitis and severe renal damage, telitacicept exhibits good therapeutic effects while also showing good safety.

[Case 7]

A patient was clinically diagnosed with ANCA-associated vasculitis (gastrointestinal vasculitis, vasculitic renal damage), comorbid with hypertension, diabetes mellitus, and hyperlipidemia, with a medical history of 9 years. The patient had successively received glucocorticoids (prednisone), glucocorticoids (prednisone) + immunosuppressants (cyclophosphamide), and glucocorticoids (methylprednisolone) + immunosuppressants (cyclophosphamide) + rituximab treatment, and the condition relapsed repeatedly several times.

Subsequently, the patient received regular hormones (prednisone acetate 7.5 mg once a day) + intermittent telitacicept (160 mg subcutaneous injection, once a week) and conventional blood pressure and blood sugar control treatment. Three months later, the erythrocyte sedimentation rate and C-reactive protein both returned to normal, and the condition was relieved.

Results showed:

① For patients who still relapsed repeatedly after treatment with glucocorticoids, glucocorticoids + immunosuppressants, or glucocorticoids + immunosuppressants + rituximab, after receiving telitacicept treatment, the erythrocyte sedimentation rate and C-reactive protein both returned to normal, and the patient's condition was relieved.

② For patients with ANCA-associated vasculitis who have a long medical history and still experience recurrent attacks after multiple regimens, telitacicept shows good therapeutic prospects.

[Case 8]

A patient was clinically diagnosed with ANCA-associated vasculitis, comorbid with systemic lupus erythematosus (involving the blood system, kidneys, lungs, serous membrane, eyes, muscles) based on multiple indicators.

The patient was treated with hormone + immunosuppressant + telitacicept: methylprednisolone (500 mg on Day 1-5, 80 mg on Day 6-15, 40 mg on Day 16-29) for anti-inflammation, cyclophosphamide (0.1 g on Day 4, 0.2 g on Day 7, 0.2 g on Day 10, 0.2 g on Day 21, 0.2 g on Day 29, cumulative 0.9 g) for immune regulation, telitacicept 160 mg subcutaneous injection once a week for a total of 3 times (Day 15, 22, and 29), plasma exchange 4 times (Day 2, 4, 7, and 10), meropenem for anti-infection, blood transfusion to correct anemia, potassium supplementation, diuresis, and other symptomatic treatments. After discharge, the patient was prescribed prednisone 50 mg / 10 tablets once a day, and telitacicept 160 mg subcutaneous injection once a week.

Results showed: For patients with ANCA comorbid with other autoimmune diseases (such as systemic lupus erythematosus nephritis involving the blood system, kidneys, lungs, serous membrane, eyes, muscles), after treatment with glucocorticoids + immunosuppressants + telitacicept, clinical symptoms improved significantly, and renal function was relatively stable. This indicates that for patients with ANCA comorbid with other autoimmune diseases, telitacicept exhibits good therapeutic potential.

[Case 9]

A patient was clinically diagnosed with ANCA-associated vasculitis based on multiple indicators. The patient had previously received postoperative treatment with hormones (methylprednisolone), hormones (prednisone) + immunosuppressants (mycophenolate mofetil capsules), and hormones (prednisone) + immunosuppressants (mycophenolate mofetil capsules combined with cyclophosphamide), and had relapsed repeatedly multiple times during the process of hormone tapering.
Later, after treatment with hormones (prednisone) + immunosuppressants (cyclophosphamide) + telitacicept (160 mg, once a week), the clinical symptoms improved significantly.
Results showed:

① For patients with ANCA-associated vasculitis who still experience recurrent attacks after treatment with glucocorticoids or glucocorticoids + immunosuppressants, telitacicept can exhibit good therapeutic prospects.

② For patients with recurrent ANCA-associated vasculitis, the effect of telitacicept is excellent.

[Case 10]

A patient was clinically diagnosed with ANCA-associated glomerulonephritis based on multiple indicators, comorbid with nephrotic syndrome, accompanied by chronic renal failure and chronic kidney disease stage 3.
After the first treatment with methylprednisolone, prednisone, and plasma exchange, the patient could not tolerate nausea, vomiting, and palpitations after hormone therapy. The hormones were gradually tapered and discontinued, leading to a relapse.
The second treatment was cyclophosphamide + mycophenolate mofetil; later, cyclophosphamide was discontinued due to cellular immune deficiency.
The third treatment was telitacicept (160 mg subcutaneous injection once a week) + mycophenolate mofetil; later, mycophenolate mofetil was discontinued due to cellular immune deficiency. The patient continued to receive telitacicept, and the administration frequency of telitacicept was adjusted based on clinical indicators.
After treatment, the patient's renal indicator serum creatinine remained stable and decreased compared with the initial hospitalization; there was no continuous progression of renal failure, and the nephropathy was effectively relieved. From massive proteinuria to moderate proteinuria, the urine protein decreased by more than 80%; serum albumin rose to the normal range.
Results showed:

① For patients with ANCA-associated vasculitis who cannot tolerate hormone + cyclophosphamide, telitacicept can be used as an ideal alternative treatment regimen, with effectiveness not inferior to hormone + cyclophosphamide, lower risk of infection, and fewer adverse events.

② Telitacicept exhibits superior safety and lower toxic and side effects compared with hormones and immunosuppressants.

In summary, compared with existing treatment regimens/means, telitacicept exhibits better safety and superior therapeutic effects in patients with ANCA-associated vasculitis. Specifically, for patients with ANCA-associated vasculitis, telitacicept shows good therapeutic prospects both in the induction phase and the maintenance phase. Furthermore, for patients who have achieved induction of remission, subsequent application of telitacicept can further improve the patient's condition, showing considerable effects. For patients who failed treatment with glucocorticoids, and/or glucocorticoids + immunosuppressants, and/or glucocorticoids + immunosuppressants + biological agents (such as rituximab), or experienced recurrent disease after treatment, telitacicept also shows good therapeutic prospects. For patients with ANCA-associated vasculitis comorbid with one or more other autoimmune diseases (including but not limited to Sjögren's syndrome, systemic lupus erythematosus, nephrotic syndrome, and lupus nephritis), telitacicept also shows good therapeutic prospects. Telitacicept exhibits better safety and superior therapeutic effects relative to glucocorticoids and/or immunosuppressants, especially in patients with severe ANCA-associated vasculitis. For patients with a long

medical history and recurrent disease after treatment, telitacicept exhibits good therapeutic potential. For patients with severe ANCA-associated vasculitis and severe renal damage, telitacicept exhibits good therapeutic effects while also showing good safety.

Example 2 Clinical Study

Primary objectives

1. To explore the effectiveness of telitacicept in the induction of remission phase for the treatment of refractory and recurrent AAV; and

2. To evaluate the effectiveness of telitacicept in the maintenance of remission treatment phase for the treatment of refractory and recurrent AAV.

Secondary objective: To preliminarily evaluate the safety of telitacicept in the treatment of refractory and recurrent ANCA-associated vasculitis.

The above descriptions merely relate to preferred implementations, which are provided as examples only and do not limit the combination of features necessary for implementing the present invention. The titles provided are not intended to limit the various implementations of the present invention. Terms such as "comprise," "contain," and "include" are not meant to be limiting. Furthermore, unless otherwise specified, reference to a singular element without a numerical modifier includes the plural form, and the terms "or" and "alternatively" mean "and/or." Unless otherwise defined herein, all technical and scientific terms used herein have the same meaning as commonly understood by a person skilled in the art.

All publications and patents mentioned in this application are incorporated herein by reference. Various modifications and variations of the described method and composition of the present invention will be apparent to a person skilled in the art without departing from the scope and spirit of the present invention. Although the present invention has been described through specific preferred embodiments, it should be understood that the claimed present invention should not be unduly limited to these specific embodiments. In fact, various variations of the described modes for carrying out the present invention that are obvious to those skilled in the relevant art are intended to be included within the scope of the appended claims.

**Claims**

1. A method for treating or alleviating ANCA-associated vasculitis, comprising administering a therapeutically effective amount of a drug targeting BLyS and/or APRIL to a patient having the ANCA-associated vasculitis.

2. The method according to claim 1, wherein the drug targeting BLyS and/or APRIL is a TACI-Fc fusion protein.

3. The method according to claim 2, wherein the TACI-Fc fusion protein comprises:

   (i) an extracellular domain of TACI or a fragment thereof binding to BLyS and/or APRIL; and
   (ii) a human immunoglobulin constant region fragment.

4. The method according to claim 3, wherein the extracellular domain of TACI or the fragment thereof binding to BLyS and/or APRIL comprises an amino acid sequence shown in SEQ ID NO: 1, SEQ ID NO: 2, or SEQ ID NO: 3.

5. The method according to claim 3, wherein a human immunoglobulin is IgG1, or the human immunoglobulin constant region fragment comprises an amino acid sequence of SEQ ID NO: 4, or comprises an amino acid sequence having at least 90%, at least 91%, at least 92%, at least 93%, at least 94%, at least 95%, at least 96%, at least 97%, at least 98%, or at least 99% identity to SEQ ID NO: 4.

6. The method according to claim 5, wherein the human immunoglobulin constant region fragment comprises amino acid modifications at 1, 2, 3, 4, 5, 6, 7, 8, or more positions compared with SEQ ID NO: 4.

7. The method according to claim 6, wherein the modification is an amino acid substitution, deletion, or insertion.

8. The method according to claim 7, wherein the substitution comprises one or more ofP3T, L8P, L14A, L15E, G17A, A110S, P111S, and/or A173T.

9. The method according to claim 7, wherein the insertion is insertion of 1, 2, 3, 4, 5, 6, 7, 8, or more amino acids at an N-terminus of the human immunoglobulin constant region fragment.

10. The method according to claim 6, wherein the human immunoglobulin constant region fragment comprises an amino acid sequence of SEQ ID NO: 5 or SEQ ID NO: 6.

11. The method according to claim 10, wherein the TACI-Fc fusion protein has an amino acid sequence having at least 75%, at least 80%, at least 85%, at least 90%, at least 95%, or at least 99% identity to SEQ ID NO: 7.

12. The method according to claim 10 or 11, wherein the TACI-Fc fusion protein has an amino acid sequence shown in SEQ ID NO: 7, SEQ ID NO: 8, or SEQ ID NO: 9.

13. The method according to claim 12, wherein the TACI-Fc fusion protein is telitacicept, atacicept, or povetacicept.

14. The method according to any one of claims 1-13, wherein the ANCA-associated vasculitis comprises, but is not limited to, one or more of granulomatosis with polyangiitis (GPA), eosinophilic granulomatosis with polyangiitis (EGPA), and microscopic polyangiitis (MPA).

15. The method according to any one of claims 1-14, comprising administering a therapeutically effective amount of a drug targeting BLyS and/or APRIL to a patient having the ANCA-associated vasculitis during an induction of remission treatment phase and/or a maintenance of remission treatment phase thereof.

16. The method according to any one of claims 1-15, wherein the patient is an adult patient or a pediatric patient.

17. The method according to any one of claims 1-16, wherein the patient with the ANCA-associated vasculitis is in a newly diagnosed disease treatment phase or a recurrent disease treatment phase.

18. The method according to any one of claims 1-17, wherein the patient with the ANCA-associated vasculitis is an inactive-phase patient, an active-phase severe patient, or an active-phase non-severe patient.

19. The method according to any one of claims 1-18, wherein the patient with the ANCA-associated vasculitis is comorbid with one or more other autoimmune diseases.

20. The method according to any one of claims 1-19, wherein the patient with the ANCA-associated vasculitis has involvement of the lungs, and/or kidneys, and/or gastrointestinal tract, and/or nervous system, and/or eyes, ears, nose, and throat, and/or systemic musculoskeletal system, and/or serous membrane, and/or blood system, and/or other tissues or organs.

21. The method according to any one of claims 1-20, wherein the patient has previously received an ANCA-associated vasculitis treatment regimen or has not previously received an ANCA-associated vasculitis treatment regimen.

22. The method according to any one of claims 1-21, wherein the ANCA-associated vasculitis treatment regimen comprises, but is not limited to, one or more of a glucocorticoid treatment regimen, an immunosuppressant treatment regimen, a biological agent treatment regimen, and plasma exchange.

23. The method according to any one of claims 1-22, comprising administering a therapeutically effective amount of a drug targeting BLyS and/or APRIL to a patient having the ANCA-associated vasculitis, while one or more of a glucocorticoid, an immunosuppressant, a biological agent, and plasma exchange are applied in combination.

24. The method according to any one of claims 1-23, wherein a single administration dose of the drug targeting BLyS and/or APRIL is about 0.1 to 10 mg/kg.

25. The method according to any one of claims 1-24, wherein the single administration dose of the drug targeting BLyS and/or APRIL is 40-240 mg, further preferably 40 mg, 60 mg, 80 mg, 100 mg, 120 mg, 140 mg, 160 mg, or 240 mg.

26. The method according to any one of claims 1-25, wherein an administration frequency of the drug targeting BLyS and/or APRIL is on-demand administration.

27. The method according to any one of claims 1-26, wherein the drug targeting BLyS and/or APRIL is used 1-8 times during each one-month interval, and/or 1-8 times during each two-month interval, and/or 1-8 times during each three-month interval, or the administration frequency is once a week, once every two weeks, once every three weeks, or once a month.

28. The method according to any one of claims 1-27, wherein an administration mode of the drug targeting BLyS and/or APRIL is subcutaneous, intramuscular, or intravenous administration, or an administration site is the thigh, abdomen, or upper arm.

FIG. 1

FIG. 2

FIG. 3

# INTERNATIONAL SEARCH REPORT

| International application No. |
| --- |
| **PCT/CN2024/103739** |

| A. | CLASSIFICATION OF SUBJECT MATTER |
| --- | --- |

A61K39/395(2006.01)i; A61P9/00(2006.01)i

According to International Patent Classification (IPC) or to both national classification and IPC

| B. | FIELDS SEARCHED |
| --- | --- |

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CNTXT, DWPI, ENTXTC, VEN, WPABSC, CNKI, 万方, WANFANG, Genbank, ISI web of Science, 中国专利生物序列检索系统, China Patent Biological Sequence Search System: 血管炎, ANCA, APRIL, BAFF, BLYS, fc融合蛋白, TACI, 申请人/发明人, Applicants/Inventors, 免疫球蛋白, TACI-fc, SEQ ID NOs: 1-9

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2022236335 A1 (ALPINE IMMUNE SCIENCES, INC.) 10 November 2022 (2022-11-10) claims 1, 25, 35 and 40, and description, paragraphs 172-173, 184-185, 224, 252 and 258, and embodiment 21 | 1-28 |
| Y | WO 2022236335 A1 (ALPINE IMMUNE SCIENCES, INC.) 10 November 2022 (2022-11-10) claims 1, 25, 35 and 40, and description, paragraphs 172-173, 184-185, 224, 252 and 258, and embodiment 21 | 11-28 |
| Y | CN 113613675 A (REMEGEN BIOPHARMACEUTICAL (YANTAI) CO., LTD.) 05 November 2021 (2021-11-05) claims 1 and 5 | 11-28 |
| Y | CN 101854951 A (ARES TRADING SA) 06 October 2010 (2010-10-06) claims 1 and 15 | 12-28 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **08 October 2024** | **15 October 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

# EP 4 748 393 A1

## INTERNATIONAL SEARCH REPORT

| International application No. |
|---|
| **PCT/CN2024/103739** |

### C.    DOCUMENTS CONSIDERED TO BE RELEVANT

| Category* | Citation of document, with indication, where appropriate, of the relevant passages | Relevant to claim No. |
|---|---|---|
| Y | CN 113573732 A (REMEGEN BIOPHARMACEUTICAL (YANTAI) CO., LTD.) 29 October 2021 (2021-10-29) <br> claims 1 and 15 | 11-28 |
| X | CN 108135976 A (BOEHRINGER INGELHEIM INTERNATIONAL GMBH et al.) 08 June 2018 (2018-06-08) <br> claims 1, 16, 29 and 40 | 1-3, 14-28 |
| X | CN 115812077 A (ALPINE IMMUNE SCIENCES INC.) 17 March 2023 (2023-03-17) <br> claims 1-119, and description, paragraphs 301-302 and 388 | 1-28 |
| X | US 2008181886 A1 (GENENTECH, INC.) 31 July 2008 (2008-07-31) <br> claims 70-89, and description, paragraph 79 | 1-3, 14-28 |
| X | US 2005163775 A1 (GENENTECH, INC.) 28 July 2005 (2005-07-28) <br> claims 1-3, 40-43 and 53, and description, paragraph 313 | 1-3, 14-28 |
| A | CN 104203977 A (NOVO NORDISK A/S) 10 December 2014 (2014-12-10) <br> description, paragraph 24 | 8, 10 |
| A | CN 101087807 A (GENENTECH INC.) 12 December 2007 (2007-12-12) <br> claims 1-127, and description, pages 45-50 | 1-4, 14-28 |
| A | US 2016333103 A1 (OSSIANIX, INC.) 17 November 2016 (2016-11-17) <br> claims 1-23, and description, paragraphs 132 and 135 | 1-28 |
| A | CN 101628111 A (ZYMOGENETICS INC.) 20 January 2010 (2010-01-20) <br> claims 1-44 | 1-28 |
| PX | Chinese SLE Treatment And Research Group. "Efficacy and Safety for Telitacicept in the Remission Maintenance Treatmentof ANCA-associated Vasculitis (TTCAZAREM)" <br> *Clinical Trials.gov ID NCT05965284*, 28 July 2023 (2023-07-28), <br> entire document | 1-28 |
| A | NAKAYAMADA, S. et al. "BAFF- and APRIL-Targeted Therapy in Systemic Autoimmune Diseases" <br> *Inflammation and Regeneration*, Vol. vol. 36, 31 December 2016 (2016-12-31), <br> text, pp. 1-6 | 1-28 |

Form PCT/ISA/210 (second sheet) (July 2022)

**INTERNATIONAL SEARCH REPORT**

International application No.

**PCT/CN2024/103739**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2024/103739**

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **1-28**
   because they relate to subject matter not required to be searched by this Authority, namely:

   Claims 1-28 set forth a method for treating or alleviating ANCA-associated vasculitis, which falls within methods for treatment of diseases as defined in PCT Rule 39.1(iv). The present report is provided on the basis of the pharmaceutical use of a targeted Blys and/or APRIL drug.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
| --- |
| **PCT/CN2024/103739** |

| Patent document cited in search report | | | Publication date (day/month/year) | Patent family member(s) | | | Publication date (day/month/year) |
| --- | --- | --- | --- | --- | --- | --- | --- |
| WO | 2022236335 | A1 | 10 November 2022 | CN | 117915937 | A | 19 April 2024 |
| | | | | US | 2024279310 | A1 | 22 August 2024 |
| | | | | EP | 4333869 | A1 | 13 March 2024 |
| | | | | CA | 3216795 | A1 | 10 November 2022 |
| | | | | AU | 2022269139 | A1 | 16 November 2023 |
| | | | | JP | 2024518163 | A | 25 April 2024 |
| | | | | KR | 20240019124 | A | 14 February 2024 |
| | | | | MX | 2023013114 | A | 17 November 2023 |
| | | | | CO | 2023016161 | A2 | 11 December 2023 |
| | | | | IL | 308336 | A | 01 January 2024 |
| | | | | PE | 20240641 | A1 | 04 April 2024 |
| | | | | CL | 2023003301 | A1 | 21 June 2024 |
| CN | 113613675 | A | 05 November 2021 | CA | 3128113 | A1 | 17 June 2021 |
| | | | | EP | 4074337 | A1 | 19 October 2022 |
| | | | | EP | 4074337 | A4 | 13 December 2023 |
| | | | | WO | 2021115321 | A1 | 17 June 2021 |
| | | | | KR | 20220054324 | A | 02 May 2022 |
| | | | | AU | 2020401160 | A1 | 19 August 2021 |
| | | | | BR | 112021025458 | A2 | 21 June 2022 |
| | | | | JP | 2022546899 | A | 10 November 2022 |
| | | | | JP | 7326500 | B2 | 15 August 2023 |
| | | | | US | 2022133633 | A1 | 05 May 2022 |
| CN | 101854951 | A | 06 October 2010 | CA | 2705357 | A1 | 22 May 2009 |
| | | | | CA | 2705357 | C | 23 October 2018 |
| | | | | WO | 2009062960 | A1 | 22 May 2009 |
| | | | | AU | 2008322930 | A1 | 22 May 2009 |
| | | | | AU | 2008322930 | B2 | 15 May 2014 |
| | | | | PT | 2219675 | E | 18 November 2013 |
| | | | | ES | 2436779 | T3 | 07 January 2014 |
| | | | | EP | 2219675 | A1 | 25 August 2010 |
| | | | | EP | 2219675 | B1 | 23 October 2013 |
| | | | | US | 2010297122 | A1 | 25 November 2010 |
| | | | | US | 8637021 | B2 | 28 January 2014 |
| | | | | JP | 2011503038 | A | 27 January 2011 |
| | | | | JP | 5469077 | B2 | 09 April 2014 |
| | | | | PL | 2219675 | T3 | 30 April 2014 |
| | | | | DK | 2219675 | T3 | 02 December 2013 |
| | | | | IL | 205716 | A0 | 30 November 2010 |
| | | | | IL | 205716 | A | 30 September 2014 |
| CN | 113573732 | A | 29 October 2021 | US | 2022002381 | A1 | 06 January 2022 |
| | | | | WO | 2021128027 | A1 | 01 July 2021 |
| | | | | EP | 3868403 | A1 | 25 August 2021 |
| | | | | EP | 3868403 | A4 | 06 April 2022 |
| | | | | CA | 3131790 | A1 | 01 July 2021 |
| | | | | AU | 2019479685 | A1 | 23 September 2021 |
| | | | | JP | 2022539785 | A | 13 September 2022 |
| | | | | KR | 20220034742 | A | 18 March 2022 |
| CN | 108135976 | A | 08 June 2018 | PH | 12018500151 | A1 | 30 July 2018 |
| | | | | CO | 2018001264 | A2 | 10 May 2018 |
| | | | | SG | 10201912593 | VA | 27 February 2020 |

Form PCT/ISA/210 (patent family annex) (July 2022)

## INTERNATIONAL SEARCH REPORT
### Information on patent family members

| International application No. |
| --- |
| **PCT/CN2024/103739** |

| Patent document cited in search report | | | Publication date (day/month/year) | Patent family member(s) | | | Publication date (day/month/year) |
| --- | --- | --- | --- | --- | --- | --- | --- |
| | | | | WO | 2017015433 | A2 | 26 January 2017 |
| | | | | WO | 2017015433 | A3 | 09 March 2017 |
| | | | | EP | 3322436 | A2 | 23 May 2018 |
| | | | | EP | 3322436 | A4 | 27 March 2019 |
| | | | | CA | 2993329 | A1 | 26 January 2017 |
| | | | | EA | 201890360 | A1 | 29 June 2018 |
| | | | | AU | 2016297575 | A1 | 15 February 2018 |
| | | | | AU | 2016297575 | B2 | 27 May 2021 |
| | | | | PE | 20180774 | A1 | 07 May 2018 |
| | | | | UA | 125433 | C2 | 09 March 2022 |
| | | | | SA | 518390788 | B1 | 26 December 2021 |
| | | | | MY | 191081 | A | 30 May 2022 |
| | | | | TW | 201712030 | A | 01 April 2017 |
| | | | | TWI | 733685 | B | 21 July 2021 |
| | | | | US | 2017022294 | A1 | 26 January 2017 |
| | | | | US | 10280231 | B2 | 07 May 2019 |
| | | | | JP | 2018527323 | A | 20 September 2018 |
| | | | | JP | 6866345 | B2 | 28 April 2021 |
| | | | | MX | 2018000959 | A | 06 June 2018 |
| | | | | CL | 2018000182 | A1 | 31 August 2018 |
| | | | | US | 2019241678 | A1 | 08 August 2019 |
| | | | | US | 10844138 | B2 | 24 November 2020 |
| | | | | TW | 202144411 | A | 01 December 2021 |
| | | | | US | 2021107998 | A1 | 15 April 2021 |
| | | | | US | 11884744 | B2 | 30 January 2024 |
| | | | | IL | 256665 | A | 28 February 2018 |
| | | | | IL | 256665 | B1 | 01 June 2023 |
| | | | | IL | 256665 | B2 | 01 October 2023 |
| | | | | BR | 112018001255 | A2 | 11 September 2018 |
| | | | | KR | 20180028522 | A | 16 March 2018 |
| | | | | KR | 102644875 | B1 | 06 March 2024 |
| CN | 115812077 | A | 17 March 2023 | TW | 202208414 | A | 01 March 2022 |
| | | | | US | 2021388054 | A1 | 16 December 2021 |
| | | | | WO | 2021226551 | A1 | 11 November 2021 |
| | | | | MX | 2022013998 | A | 16 February 2023 |
| | | | | JP | 2023525032 | A | 14 June 2023 |
| | | | | PE | 20230494 | A1 | 23 March 2023 |
| | | | | CL | 2022003104 | A1 | 14 July 2023 |
| | | | | CO | 2022017780 | A2 | 17 March 2023 |
| | | | | WO | 2021226553 | A2 | 11 November 2021 |
| | | | | WO | 2021226553 | A3 | 28 April 2022 |
| | | | | EP | 4146684 | A2 | 15 March 2023 |
| | | | | CA | 3178882 | A1 | 11 November 2021 |
| | | | | BR | 112022022433 | A2 | 13 December 2022 |
| | | | | AU | 2021267276 | A1 | 15 December 2022 |
| | | | | US | 2023241168 | A1 | 03 August 2023 |
| | | | | KR | 20230029621 | A | 03 March 2023 |
| | | | | IL | 297980 | A | 01 January 2023 |
| | | | | MX | 2022013999 | A | 16 February 2023 |
| | | | | JP | 2023525033 | A | 14 June 2023 |

Form PCT/ISA/210 (patent family annex) (July 2022)

| INTERNATIONAL SEARCH REPORT Information on patent family members | | | | International application No. PCT/CN2024/103739 | | |
|---|---|---|---|---|---|---|

| Patent document cited in search report | | | Publication date (day/month/year) | Patent family member(s) | | | Publication date (day/month/year) |
|---|---|---|---|---|---|---|---|
| | | | | BR | 112022022524 | A2 | 13 December 2022 |
| | | | | US | 2024218045 | A1 | 04 July 2024 |
| | | | | EP | 4146683 | A1 | 15 March 2023 |
| | | | | CA | 3178885 | A1 | 11 November 2021 |
| | | | | IL | 297981 | A | 01 January 2023 |
| | | | | AU | 2021268033 | A1 | 15 December 2022 |
| | | | | US | 2021363223 | A1 | 25 November 2021 |
| | | | | US | 11274140 | B2 | 15 March 2022 |
| | | | | KR | 20230029622 | A | 03 March 2023 |
| US | 2008181886 | A1 | 31 July 2008 | WO | 2006052493 | A1 | 18 May 2006 |
| | | | | CA | 2585927 | A1 | 18 May 2006 |
| | | | | AU | 2005305182 | A1 | 18 May 2006 |
| | | | | MX | 2007005378 | A | 14 February 2008 |
| | | | | EP | 1812472 | A1 | 01 August 2007 |
| US | 2005163775 | A1 | 28 July 2005 | MX | 2007007649 | A | 18 October 2007 |
| | | | | WO | 2006068867 | A1 | 29 June 2006 |
| | | | | WO | 2006068867 | A9 | 02 August 2007 |
| | | | | AU | 2005319485 | A1 | 29 June 2006 |
| | | | | EP | 1831253 | A1 | 12 September 2007 |
| | | | | CA | 2590936 | A1 | 29 June 2006 |
| | | | | JP | 2008525449 | A | 17 July 2008 |
| CN | 104203977 | A | 10 December 2014 | LT | 2814842 | T | 12 November 2018 |
| | | | | HRP | 20181817 | T1 | 28 December 2018 |
| | | | | ES | 2690786 | T3 | 22 November 2018 |
| | | | | EP | 2814842 | A1 | 24 December 2014 |
| | | | | EP | 2814842 | B1 | 22 August 2018 |
| | | | | EP | 3456742 | A1 | 20 March 2019 |
| | | | | RS | 57827 | B1 | 31 December 2018 |
| | | | | PL | 2814842 | T3 | 31 December 2018 |
| | | | | US | 2019119367 | A1 | 25 April 2019 |
| | | | | US | 10906965 | B2 | 02 February 2021 |
| | | | | DK | 2814842 | T3 | 10 December 2018 |
| | | | | US | 2018016326 | A1 | 18 January 2018 |
| | | | | US | 10150809 | B2 | 11 December 2018 |
| | | | | SI | 2814842 | T1 | 30 October 2018 |
| | | | | JP | 2015508763 | A | 23 March 2015 |
| | | | | JP | 6400480 | B2 | 03 October 2018 |
| | | | | US | 2015018528 | A1 | 15 January 2015 |
| | | | | US | 9663568 | B2 | 30 May 2017 |
| | | | | WO | 2013120554 | A1 | 22 August 2013 |
| CN | 101087807 | A | 12 December 2007 | TW | 200628169 | A | 16 August 2006 |
| | | | | CA | 2580271 | A1 | 20 April 2006 |
| | | | | ZA | 200702335 | B | 27 May 2009 |
| | | | | PA | 8647601 | A1 | 17 January 2007 |
| | | | | BRPI | 0516297 | A | 02 September 2008 |
| | | | | US | 2006110387 | A1 | 25 May 2006 |
| | | | | GT | 200500276 | A | 08 May 2006 |
| | | | | PE | 20060934 | A1 | 11 October 2006 |
| | | | | RU | 2007116980 | A | 20 November 2008 |
| | | | | RU | 2411956 | C2 | 20 February 2011 |

Form PCT/ISA/210 (patent family annex) (July 2022)

## INTERNATIONAL SEARCH REPORT
### Information on patent family members

International application No.

**PCT/CN2024/103739**

| Patent document cited in search report | | | Publication date (day/month/year) | Patent family member(s) | | | Publication date (day/month/year) |
|---|---|---|---|---|---|---|---|
| | | | | NO | 20072312 | L | 05 July 2007 |
| | | | | US | 2007025987 | A1 | 01 February 2007 |
| | | | | ZA | 200804339 | B | 31 March 2010 |
| | | | | KR | 20070100228 | A | 10 October 2007 |
| | | | | AR | 052219 | A1 | 07 March 2007 |
| | | | | WO | 2006041680 | A2 | 20 April 2006 |
| | | | | WO | 2006041680 | A3 | 23 August 2007 |
| | | | | SV | 2006002256 | A | 20 April 2006 |
| | | | | EP | 1812060 | A2 | 01 August 2007 |
| | | | | AU | 2005294666 | A1 | 20 April 2006 |
| | | | | IL | 181922 | A0 | 13 April 2008 |
| | | | | SG | 165344 | A1 | 28 October 2010 |
| | | | | JP | 2008515890 | A | 15 May 2008 |
| US | 2016333103 | A1 | 17 November 2016 | US | 10435474 | B2 | 08 October 2019 |
| | | | | US | 2020024354 | A1 | 23 January 2020 |
| | | | | US | 11267894 | B2 | 08 March 2022 |
| | | | | WO | 2015100246 | A1 | 02 July 2015 |
| CN | 101628111 | A | 20 January 2010 | HRP | 20030948 | A2 | 30 June 2004 |
| | | | | HRP | 20030948 | B1 | 30 June 2013 |
| | | | | SI | 1436003 | T1 | 26 February 2010 |
| | | | | US | 2011229473 | A1 | 22 September 2011 |
| | | | | US | 8524232 | B2 | 03 September 2013 |
| | | | | EA | 200600894 | A1 | 31 August 2007 |
| | | | | EA | 010594 | B1 | 30 October 2008 |
| | | | | MXPA | 03010687 | A | 01 July 2004 |
| | | | | DK | 1436003 | T3 | 15 March 2010 |
| | | | | US | 2003103986 | A1 | 05 June 2003 |
| | | | | EA | 200301146 | A2 | 24 June 2004 |
| | | | | EA | 200301146 | A3 | 24 February 2005 |
| | | | | EA | 007275 | B1 | 25 August 2006 |
| | | | | IL | 158920 | A0 | 12 May 2004 |
| | | | | NO | 20035173 | D0 | 21 November 2003 |
| | | | | NO | 20035173 | L | 23 January 2004 |
| | | | | NO | 337295 | B1 | 07 March 2016 |
| | | | | US | 2006034852 | A1 | 16 February 2006 |
| | | | | US | 7501497 | B2 | 10 March 2009 |
| | | | | KR | 20090080570 | A | 24 July 2009 |
| | | | | KR | 100976743 | B1 | 19 August 2010 |
| | | | | EP | 1436003 | A2 | 14 July 2004 |
| | | | | EP | 1436003 | A4 | 27 April 2005 |
| | | | | EP | 1436003 | B1 | 28 October 2009 |
| | | | | EP | 1436003 | B3 | 14 March 2012 |
| | | | | SI | 2116259 | T1 | 30 November 2012 |
| | | | | ES | 2334772 | T3 | 16 March 2010 |
| | | | | ES | 2334772 | T7 | 19 November 2012 |
| | | | | UA | 82830 | C2 | 26 May 2008 |
| | | | | US | 2010183609 | A1 | 22 July 2010 |
| | | | | US | 7964711 | B2 | 21 June 2011 |
| | | | | JP | 2009232856 | A | 15 October 2009 |
| | | | | JP | 5149245 | B2 | 20 February 2013 |

Form PCT/ISA/210 (patent family annex) (July 2022)

**INTERNATIONAL SEARCH REPORT**
Information on patent family members

International application No.

**PCT/CN2024/103739**

| Patent document cited in search report | Publication date (day/month/year) | Patent family member(s) | | Publication date (day/month/year) |
|---|---|---|---|---|
| | | HK | 1137659 A1 | 06 August 2010 |
| | | KR | 20040030628 A | 09 April 2004 |
| | | PL | 366760 A1 | 07 February 2005 |
| | | PL | 219013 B1 | 27 February 2015 |
| | | US | 2010129384 A1 | 27 May 2010 |
| | | US | 7862814 B2 | 04 January 2011 |
| | | ZA | 200308984 B | 20 July 2004 |
| | | HRP | 20130413 A2 | 31 July 2013 |
| | | US | 2014328844 A1 | 06 November 2014 |
| | | US | 9346878 B2 | 24 May 2016 |
| | | YU | 92503 A | 25 May 2006 |
| | | RS | 52228 B | 31 October 2012 |
| | | ES | 2379977 T3 | 07 May 2012 |
| | | EP | 2116259 A1 | 11 November 2009 |
| | | EP | 2116259 B1 | 25 January 2012 |
| | | IL | 217265 A0 | 29 February 2012 |
| | | IL | 217265 A | 30 May 2013 |
| | | MEP | 21708 A | 10 June 2010 |
| | | CY | 1109751 T1 | 10 September 2014 |
| | | AU | 2002305646 B2 | 04 September 2008 |
| | | AU | 2002305646 C1 | 06 August 2009 |
| | | PT | 1436003 E | 12 March 2010 |
| | | US | 2009209006 A1 | 20 August 2009 |
| | | US | 7635767 B2 | 22 December 2009 |
| | | CA | 2448123 A1 | 28 November 2002 |
| | | CA | 2448123 C | 11 September 2012 |
| | | DK | 2116259 T3 | 21 May 2012 |
| | | WO | 02094852 A2 | 28 November 2002 |
| | | WO | 02094852 A8 | 22 April 2004 |
| | | RS | 20120253 A1 | 28 February 2013 |
| | | BR | 0209933 A | 30 March 2004 |
| | | BRPI | 0209933 B1 | 16 October 2018 |
| | | BRPI | 0209933 B8 | 25 May 2021 |
| | | JP | 2004535182 A | 25 November 2004 |
| | | HK | 1076603 A1 | 20 January 2006 |
| | | DE | 60234202 D1 | 10 December 2009 |
| | | CY | 1112840 T1 | 10 February 2016 |
| | | ATE | 446771 T1 | 15 November 2009 |
| | | ATE | 542545 T1 | 15 February 2012 |
| | | PL | 403488 A1 | 08 July 2013 |
| | | US | 2010130728 A1 | 27 May 2010 |
| | | US | 7951919 B2 | 31 May 2011 |
| | | KR | 20100061860 A | 09 June 2010 |
| | | KR | 101021124 B1 | 14 March 2011 |
| | | PT | 2116259 E | 09 April 2012 |
| | | NZ | 529638 A | 31 August 2007 |
| | | US | 2013309231 A1 | 21 November 2013 |
| | | US | 8815238 B2 | 26 August 2014 |

Form PCT/ISA/210 (patent family annex) (July 2022)

REFERENCES CITED IN THE DESCRIPTION

**Patent documents cited in the description**

- US 5969102 A
- US 6316222 B
- US 6500428 B
- US 569245 A
- US 09627206 B
- CN 101323643 A

**Non-patent literature cited in the description**

- **GAPUD E J** ; **SEO P** ; **ANTIOCHOS B.** ANCA-associated vasculitis pathogenesis: a commentary[J. *Current rheumatology reports*, 2017, vol. 19, 1-7
- **LIU L** ; **LU H** ; **ZOU G et al.** Efficacy and safety of low-dose rituximab as induction therapy for antineutrophil cytoplasmic antibody-associated vasculitis with renal involvement: a Chinese case series. *BMC Nephrol*, 08 February 2023, vol. 24 (1), 28

- **CHUNG, S. A.** ; **LANGFORD, C. A.** ; **MAZ, M.** ; **ABRIL, A.** ; **GORELIK, M.** ; **GUYATT, G.** ; **MUSTAFA, R. A**. 2021 American College of Rheumatology/Vasculitis Foundation Guideline for the Management of Antineutrophil Cytoplasmic Antibody-Associated Vasculitis. *Arthritis Care & Research*, 2021, vol. 73 (8), 1088-1105
- **DAVID** ; **JAYNE** ; **DANIEL et al.** Efficacy and Safety of Belimumab and Azathioprine for Maintenance of Remission in Antineutrophil Cytoplasmic Antibody-Associated Vasculitis: A Randomized Controlled Study. *Arthritis & rheumatology (Hoboken, N.J.)*, 2019
- *J. Biol. Chem.*, 1968, vol. 243, 3558